Europäisches Patentamt

European Patent Office

Office européen des brevets

(19)

(11) Veröffentlichungsnummer: **0 016 946**
**A1**

(12) # EUROPÄISCHE PATENTANMELDUNG

(21) Anmeldenummer: 80100840.0

(22) Anmeldetag: 20.02.80

(51) Int. Cl.³: **C 12 N 9/18,** C 12 N 9/96, C 12 Q 1/60

(30) Priorität: 22.03.79 DE 2911284

(43) Veröffentlichungstag der Anmeldung: 15.10.80
Patentblatt 80/21

(84) Benannte Vertragsstaaten: **AT BE CH DE FR GB IT LU NL SE**

(71) Anmelder: **BOEHRINGER MANNHEIM GMBH,
Sandhofer Strasse 112-132 Postfach 31 01 20,
D-6800 Mannheim 31-Waldhof (DE)**

(72) Erfinder: **Klose, Sigmar, Dr., Maxhöhe 17, D-8131 Berg
(DE)**
Erfinder: **Buschek, Herbert, Possenhofener Strasse 12,
D-8130 Starnberg (DE)**
Erfinder: **Schlumberger, Helmut,
Kaiser-Heinrich-Strasse 23, D-8121 Polling (DE)**

(74) Vertreter: **Weickmann, Heinrich, Dipl.-Ing Patentanwälte
Dipl.Ing.H.Weickmann et al, Dipl.Phys.Dr.K.Fincke
Dipl.Ing.F.A.Weickmann Dipl.Chem.B.Huber,
Dr.-Ing.H.Liska Möhlstrasse 22, D-8000 München 86 (DE)**

(54) Verfahren und Reagenz zur Aktivierung der Cholesterinesterase.

(57) Die Aktivierung der Cholesterinesterase in ionenhaltiger Lösung erfolgt durch Zusatz von einem Polyäthoxyäther oder -ester oder/und einer Gallensäureverbindung und/oder Salzen von Fettsäuren mit 6 bis 10 Kohlenstoffatomen in Kombination mit einem synergistisch wirkenden Alkohol, der aus den geradkettigen, verzweigten oder cyclischen aliphatischen Alkoholen mit 5 bis 12 Kohlenstoffatomen, gegebenenfalls halogensubstituierten, aromatischen Alkoholen und den mit mehreren Halogenatomen substituierten aliphatischen Alkoholen mit 1 bis 3 Kohlenstoffatomen ausgewählt ist.

- 1 -

Verfahren und Reagenz zur Aktivierung der Cholesterinesterase

Die Erfindung betrifft ein Verfahren und ein Mittel zur Aktivierung von Cholesterinesterase, insbesondere bei der enzymatischen Umsetzung von Estern in Reaktionsgemischen. Solche Reaktionsgemische werden unter anderem in der klinisch-chemischen Analytik und in der lebensmittelchemischen Analytik verwendet.

Bekannte Probleme bei der Konzipierung von cholesterinesterabhängigen Tests sind die Einsparung von teurem Enzym, Erzielung höherer Reaktionsgeschwindigkeiten und möglichst freie Wählbarkeit von Art und Gehalt der Detergentien und Puffer nach übergeordneten Gesichtspunkten.

In einem gepufferten Medium, das keine weiteren Komponenten enthält, entwickelt die Cholesterinesterase gegenüber ihren Substraten keine oder nur eine sehr geringe Aktivität. Es ist daher erforderlich, aktivierende Zusätze für das Enzym zu finden, durch welche die Esterspaltung gesteigert wird. Als solche aktivierenden Zusätze sind einige Detergentien bekannt (US-PS 3 884 764, DE-OS 24 09 696, DE-OS 25 12 605, DE-OS 25 12 585, DE-PS 2 506 712, Biochim. Biophys. Acta 270 (1972) 156-166;

0016946

z. B. Gallensäuren, Triton X-1oo, Thesit).

Ein höherer Salzgehalt des Mediums unterstützt dessen aktivierende Wirkung. Nicht alle Puffersysteme sind jedoch in gleicher Weise geeignet. Gleiches gilt für die Detergentien. Man findet hier unter bestimmten Bedingungen solche mit guter und weniger guter Wirkung bis hin zu solchen, die überhaupt keine aktivierende Wirkung zeigen. Welches Detergens in dieser Hinsicht geeignet ist, läßt sich meist nicht vorhersagen.

Den aktivierenden Detergentien ist gemeinsam, daß sie in einem vorgegebenen Medium in einem bestimmten Konzentrationsbereich ihre optimal aktivierende Wirkung entfalten. Die Optima können in ungünstig hohen Konzentrationsbereichen liegen. Außerhalb der Optima wird ein höherer Einsatz des verhältnismäßig teuren Enzyms nicht in optimaler Weise in höhere Reaktionsgeschwindigkeiten umgesetzt.

Außerdem lassen sich Aktivitätssteigerungen durch Optimierung des Detergensgehalts und Steigerung der Ionenstärke nur in relativ engem Rahmen erzielen.

Da die in Betracht kommenden Reaktionsgemische in der Regel noch weitere Enzyme und Substrate für die Folge- und Indikatorreaktionen enthalten, ist bei der Auswahl des Puffers und des Detergens sowie bei der Optimierung der jeweilig einzusetzenden Mengen keine freie Wählbarkeit gegeben.

Bei der Festlegung der Reaktionsbedingungen sind vielmehr immer die Wechselwirkungen aller Reaktionspartner untereinander, insbesondere mögliche Hemmungen oder nachteilige Stabilitätsbeeinflussungen anderer Enzyme durch Detergentien oder/und hohe Ionenstärken zu berücksichti-

0016946

gen.

Weiterhin sind bei der Konzipierung eines Testsystems Löslichkeitsgrenzen und Probleme der Galenik zu beachten.

Im letzteren Falle lassen sich die häufig flüssigen bis wachsartigen Detergentien selten in der zur Aktivierung benötigten Menge in Feststoffgemischen, die ja trocken und rieselfähig sein sollen, unterbringen. Dies ist besonders bei Eintopfreagenzien von Bedeutung.

Schließlich ist noch zu berücksichtigten, daß die meisten in Frage kommenden Detergentien nicht als definierte Verbindungen, sondern als Stoffgemische vorliegen. Folglich treten Chargenunterschiede auf, die sich auch bei der aktivierenden Wirkung bemerkbar machen. Es kann sich daher sehr nachteilig auswirken, wenn die Aktivierung allein von einem Detergens abhängig ist.

Infolgedessen besteht ein erheblicher Bedarf an weiteren und besseren Mitteln und Verfahren zur Aktivierung der Cholesterinesterase.

Der Erfindung liegt die Aufgabe zugrunde, die oben erwähnten Nachteile zu beseitigen und ein Verfahren und ein Mittel der genannten Art zu schaffen, bei dem Cholesterinesterase eingespart wird, unter bestimmten Bedingungen höhere Reaktionsgeschwindigkeiten erzielt werden können, mit geringerer Ionenstärke gearbeitet werden kann, Detergentien nur in möglichst minimaler Konzentration benötigt werden und auch solche Detergentien verwendbar werden, die alleine nur eine schlechte oder überhaupt keine aktivierende Wirkung zeigen.

Gelöst wird diese Aufgabe erfindungsgemäß durch ein Verfahren zur Aktivierung von Cholesterinesterase in ionenhaltigen Lösungen durch Zusatz von Detergentien, welches dadurch gekennzeichnet ist, daß man

a) als oberflächenaktives Mittel einen Polyäthoxyäther oder -ester oder/und eine Gallensäureverbindung und/ oder Salze von Fettsäuren mit 6 bis 1o Kohlenstoffatomen in Kombination mit

b) einem synergistisch wirkenden Alkohol, der aus den geradkettigen, verzweigten oder cyclischen aliphatischen Alkoholen mit 5 bis 12 Kohlenstoffatomen, gegebenenfalls halogensubstituierten, aromatischen Alkoholen und den mit mehreren Halogenatomen substituierten aliphatischen Alkoholen mit 1 bis 3 Kohlenstoffatomen ausgewählt ist,

zusetzt.

Die Erfindung beruht auf der überraschenden Feststellung der Tatsache, daß die genannten Alkohole, die für sich allein keine aktivierende Wirkung auf die Cholesterinesterase zeigen, die an sich bekannte aktivierende Wirkung bestimmter Detergentien synergistisch deutlich verstärken und bei bestimmten, für sich alleine nicht aktivierenden Detergentien eine Aktivierung bewirken.

Dies führt dazu, daß derartige nicht aktivierende, im Handel weit verbreitete Detergentien eingesetzt werden können. Im gleichen Sinne werden wegen ihrer definierten Molekülstruktur und festen Konsistenz erwünschte, aber wegen schlechter aktivierender Eigenschaften bisher ungeeignete ionische Detergentien verwendbar. Weiterhin läßt sich Enzym einsparen, die maximal erreichbare Reaktionsgeschwindigkeit erhöhen, die erforderliche Ionenstärke erniedrigen und schließlich durch die vielfältigen Kombinationsmöglichkeiten das esteraseaktivierende System den durch weitere Komponenten des Reaktionsgemisches diktierten Bedingungen anpassen.

Beispiele für im Rahmen der Erfindung geeignete Polyäthoxyäther sind Fettalkoholpolyglykoläther, wie Polyoxyäthylenlauryläther und Hydroxypolyäthoxydodekan, Alkylaryläther und Arylalkyläther, wie Polyoxyäthylen-nonylphenyläther und Polyoxyäthylen-octylphenyläther, Fettsäureester, wie Polyoxyäthylensorbitan-monolaurat und ähnliche. Bei den Gallensäureverbindungen werden Cholsäure, Desoxycholsäure und deren Alkalisalze bevorzugt. Desgleichen werden unter den Fettsäuresalzen ebenfalls die Alkalisalze, ganz besonders die Natriumsalze, bevorzugt.

Typische Beispiele für im Rahmen der Erfindung geeignete geradkettige, verzweigte oder cyclische aliphatische Alkohole sind Pentanol, tert.-Amylalkohol, Methylpentanol, Hexanol, Cyclohexanol, Octanol, Isooctanol, Dekanol und Dodekanol. Beispiele für geeignete aromatische Alkohole sind Phenol und Dihydroxynaphthol. Bevorzugt werden die halogensubstituierten Phenole und Naphthole, wie die Mono-, Di- und Trichlorphenole. Als mehrfach halogensubstituierte aliphatische Alkohole können beispielsweise Dichloräthanol, Trichloräthanol, Dichlormethanol, Trichlormethanol, Trichlorpropanol, Tetrachlorpropanol usw. verwendet werden. Als Halogensubstituenten kommen bei den aromatischen und aliphatischen Alkoholen Chlor, Brom und Fluor in Betracht, bevorzugt wird Chlor. Die aromatischen Alkohole können auch eine oder zwei Alkylgruppen mit 1 bis 2 C-Atomen aufweisen.

Welcher dieser Alkohole (im folgenden Adjuvans genannt) für einen bestimmten Fall besonders geeignet ist, läßt sich leicht durch Vorversuche ermitteln. Meist sind Bedingungen, wie Art und Stärke des Puffersystems, Art und Konzentration des Detergens sowie Haltbarkeit und gewünschte Konsistenz der Aufbewahrungsform des Reaktionsgemisches vorgegeben. Diesen Bedingungen kann dann durch Auswahl des geeigneten Adjuvans und Anpassung der einzusetzenden Menge entsprochen werden. Im allgemeinen wurde gefunden, daß Konzentrationen zwischen 1 und 3oo mM Adjuvans/l gute Ergebnisse bringen.

Bevorzugt werden die Alkohole in Mengen zwischen o,o1 und 5 %, Volumen-% bei flüssigen Alkoholen, Gew.-% bei festen Alkoholen, eingesetzt. Besonders bevorzugt sind o,o5 bis 3 %.

In den Polyäthoxy-Derivaten enthalten die Alkylgruppen im allgemeinen 8 bis 22 C-Atome, die Aralkyl- und Alkylarylgruppen enthalten vorzugsweise einen Phenylrest. Der Glykolrest dieser Detergentien weist im allgemeinen 3 bis 25 Oxyäthylengruppen auf.

Die Detergentien werden zweckmäßig in einer Menge zwischen o,o5 und 5 Vol.-%, bzw. Gew.-% bei festen Substanzen, eingesetzt. Bevorzugt werden o,1 bis 3 %.

Ein weiterer Gegenstand der Erfindung ist ein Mittel zur Aktivierung der Cholesterinesterase in ionenhaltiger Lösung, enthaltend wenigstens ein oberflächenaktives Mittel auf Basis eines Polyäthoxyäthers oder -esters oder/ und einer Gallensäureverbindung und/oder Salze von Fettsäuren mit 6 bis 1o Kohlenstoffatomen, welches gekennzeichnet ist durch einen Gehalt an synergistisch wirkendem Alkohol, der aus den geradkettigen Alkoholen oder verzweigtkettigen oder cyclischen aliphatischen Alkoholen mit 5 bis 12 Kohlenstoffatomen, gegebenenfalls halogensubstituierten, aromatischen Alkoholen, halogensubstituier-

0016946

ten aliphatischen Alkoholen mit 1 bis 3 Kohlenstoffatomen ausgewählt ist.

Die Erfindung ist anwendbar im Zusammenhang mit allen Arten von Messungen und Reaktionen, bei denen Cholesterinesterase in Reaktionsgemischen verwendet wird. Derartige Reaktionsgemische sind dem Fachmann allgemein bekannt und werden, soweit sie für analytische Zwecke bestimmt sind, beispielsweise in "Methoden der enzymatischen Analyse" H.U. Bergmeyer, Verlag Chemie Weinheim, . Bergstraße beschrieben. Eine nähere Erläuterung derartiger Reaktionsgemische erübrigt sich daher hier. Durch die Erfindung wird es möglich, Enzyme einzusparen, unter gegebenen Bedingungen höhere Reaktionsgeschwindigkeiten zu erzielen, Puffer geringerer Ionenstärke einzusetzen, Detergentien in geringerer Konzentration zu verwenden und auch solche Detergentien anwendbar zu machen, die alleine nur eine ungenügende oder überhaupt keine aktivierende Wirkung zeigen. Die Erfindung eignet sich für Cholesterinesterase aus Mikroorganismus oder anderen Ursprungs, wie aus Pankreas oder Leber.

Die folgenden Beispiele erläutern die Erfindung weiter in Verbindung mit der beigefügten Zeichnung. In dieser stellen dar:

Fig. 1 eine schematische Darstellung der verwendeten Apparatur zur Bestimmung der Cholesterinesteraseaktivität,

Fig. 2 eine graphische Darstellung der bei der angewendeten Methode auftretenden Extinktionsänderung pro Zeiteinheit, die zur Ermittlung der Geschwindigkeit der Esterspaltung verwendet wurde.

Folgende Tabelle zeigt die chemische Zusammensetzung der mit Handelsnamen bezeichneten Detergentien:

| Brij 35 | Polyoxyäthylen-lauryläther |
|---|---|
| Genapol OX-1oo | Fettalkohol-polyglykoläther |
| Tergitol NPX | Polyoxyäthylen-nonylphenyläther (ca. 1o,5 Oxyäthylengruppen) |
| Thesit | Hydroxypolyäthoxy-dodekan |
| Triton X-1oo | Polyäthoxy-octylphenyläther (9 bis 1o Oxyäthylengruppen) |
| Tween 2o | Polyoxyäthylen-sorbitan-mono-laurat. |

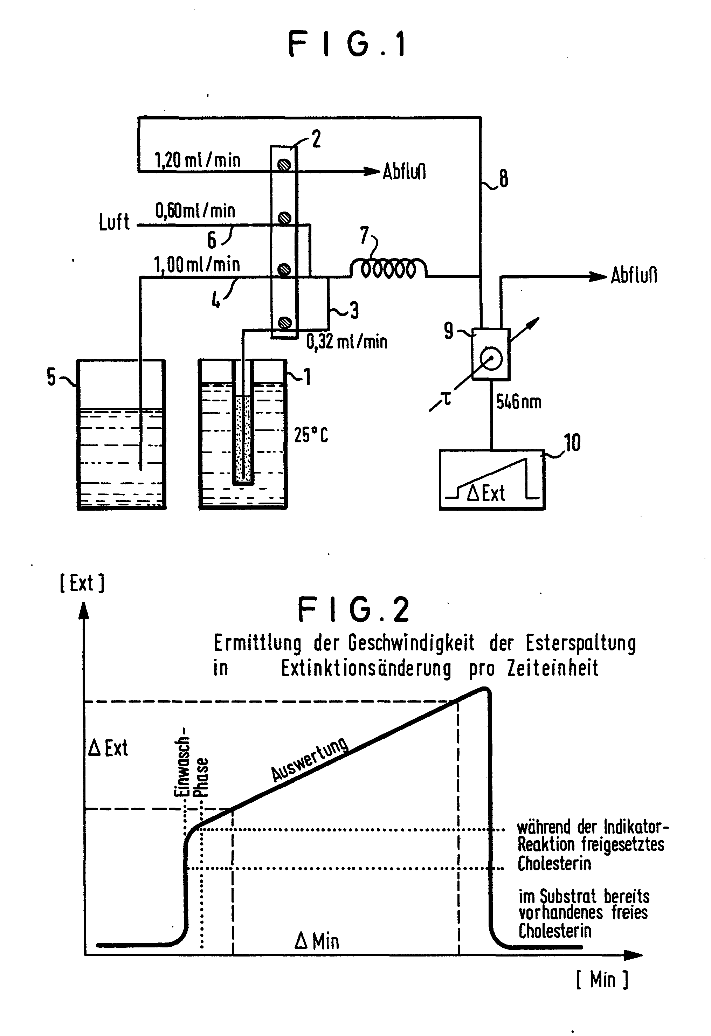

Zum Nachweis der aktivierenden Wirkung der zu untersuchenden synergistischen Kombinationen der Erfindung auf Cholesterinesterase wurde die in Fig. 1 dargestellte Apparatur verwendet.

Aus einem temperierbaren Reaktionsgefäß 1 (25°C), in dem sich die Esterspaltung vollzieht, werden über eine Schlauchpumpe 2 durch einen Förderschlauch 3 definierten Querschnitts kontinuierlich o,32 ml Gemisch/Minute entnommen. Über die gleiche Pumpe wird durch einen zweiten Schlauch 4 aus einem Vorratsgefäß 5 ebenfalls kontinuierlich 1,o ml Indikatorlösung/Minute gefördert, hinter der Pumpe 2 mit einem Luftstrom 6 von konstant o,6 ml Luft/Minute zusammengeführt und damit segmentiert und dann mit dem zuvor beschriebenen Reaktionsgemischstrom vereinigt. Der Gesamtstrom passiert daraufhin zwei Glasschlangen 7, wodurch eine Durchmischung der Flüssigkeiten und die notwendige Reaktionszeit für die Indikatorreaktion bis zum Endpunkt gewährleistet wird. Durch Abziehen eines Stroms von 1,2 ml/Minute unmittelbar vor der Küvette durch Schlauchleitung 8 senkrecht nach oben werden die Luftblasen wieder entfernt. Der nicht segmentierte Reststrom wird zur photometrischen Messung (bei

0016946

546 nm) in eine Durchflußküvette (1 cm Schichtdicke) geleitet und die Meßwerte werden vom Schreiber 1o registriert.

Wegen der gleichbleibenden Bedingungen bezüglich der Indikatorreaktion sind Änderungen in Höhe und Verlauf des Meßsignals allein von den Vorgängen im Reaktionsgefäß abhängig. Findet dort eine Esterspaltung statt, so bewirkt deren kinetischer Verlauf eine Zunahme des Meßsignals, welches als positive Steigung mit dem Schreiber 1o registriert wird. Die Steilheit des Anstiegs ist ein Maß für die Reaktionsgeschwindigkeit und damit für die Cholesterinesteraseaktivität im Reaktionsansatz.

Wegen des getrennten Ablaufs von Esterspaltung und Indikatorreaktion ist eine gegenseitige Beeinflussung ausgeschlossen. Natürlich wären auch andere Indikatorsysteme einschließlich der Sauerstoffelektrode verwendbar.

Indikatorlösung:
o,1 M Kaliumphosphatpuffer, pH 7,o
1,5 mM/l 4-Aminoantipyrin
5 mM/l Phenol
3 ‰ Hydroxypolyäthoxydodekan
8 U/ml Peroxidase
1 U/ml Cholesterinoxidase

Die Indikatorlösung setzt zunächst freies Cholesterin zu Cholestenon und $H_2O_2$ um. Das entstandene $H_2O_2$ wird mit Phenol und 4-Aminoantipyrin zu einem roten Farbstoff umgesetzt, dessen Absorption photometrisch gemessen wird. Die Menge des entstehenden Farbstoffs ist der Konzentration des freien Cholesterins proportional. Die Konzentration an freiem Cholesterin setzt sich aus dem bereits im Substrat vorhandenen Anteil an freiem Cholesterin sowie aus dem durch die Esterase freigesetzten Cholesterin zusammen.

Ansatz im Reaktionsgefäß:

o,5 ml Substrat (Kontrollserum; 36o mg Gesamtcholeste-
rin/dl)

Detergens, je nach Versuch

Adjuvans, je nach Versuch

Puffer auf 5 ml.

Start mit 5o ul einer Esterase-Stocklösung, woraufhin o,oo3 U/ml Esterase im Ansatz vorliegen. Bei Aktivitätsabstufungen wurde diese Menge variiert.

Nach erfolgtem Start und Durchmischung wird bei laufender Pumpe 2 der Förderschlauch 3 in die Lösung im Reaktionsgefäß 1 für etwa 1o Minuten eingetaucht. Anschließend wird zur besseren Trennung der Signale in jeweils etwa 1o Segmenten wechselweise Luft und bidest. Wasser angesaugt. Daraufhin kann die nächste Bestimmung erfolgen.

Die nachstehenden Beispiele zeigen die durch die Erfindung ermöglichte Einsparung an Enzym (a), die Erzielung höherer Reaktionsgeschwindigkeiten unter vorgegebenen Bedingungen (b), die Verwendung von Puffern geringerer Ionenstärke (c), den Einsatz von Detergentien in geringerer Konzentration (d) und die Verwendung auch solcher Detergentien, die unter bestimmten Bedingungen nur eine ungenügende oder überhaupt keine aktivierende Wirkung zeigen (e).

a) Einsparung von Cholesterinesterase

Die Möglichkeit der Einsparung von Cholesterinesterase wird wie folgt aufgezeigt:

0016946

Zunächst wird eine Enzymaktivitätsabstufung in Abwesenheit von Adjuvantien vorgenommen. Die Vergleichsmessungen erfolgen dann unter Zusatz von Adjuvantien bei einem Cholesterinesterase-Einsatz von o,oo3 U/ml. Aufgrund der aufgezeigten Beziehung zwischen eingesetzter Aktivität und resultierender Geschwindigkeit wird dann die erzielte Aktivitätssteigerung ermittelt.

B e i s p i e l   1

o,1 M Tris/Weinsäure-Puffer, pH 8,o
5 %o Triton X-1oo

| Eingesetzte Aktivität | Resultierende Geschwindigkeit |
|---|---|
| o U/ml | o Ext/1o Minuten |
| o,oo12 | o,o12 |
| o,oo3o | o,o32 |
| o,oo45 | o,o52 |
| o,oo6o | o,o58 |
| o,oo89 | o,o94 |

1.1   Zusatz von 2 Vol.-% tert.-Amylalkohol

| | |
|---|---|
| Eingesetzte Aktivität | o,oo3o U/ml |
| Erzielte Geschwindigkeit | o,o46 Ext/1o Min. |
| Erforderlicher Enzymeinsatz für diese Geschwindigkeit ohne Adjuvans | o,oo44 U/ml |
| Erzielte Aktivität | 147 % |

1.2   Zusatz von o,4 Vol.-% 4-Methylpentanol-(2)

| | |
|---|---|
| Eingesetzte Aktivität | o,oo3 U/ml |
| Erzielte Geschwindigkeit | o,o65 Ext/1o Min. |
| Erforderlicher Enzymeinsatz für diese Geschwindigkeit ohne Adjuvans | o,oo63 U/ml |
| Erzielte Aktivität | 21o % |

1.3   Zusatz von 1 Vol.-% Cyclohexanol

Eingesetzte Aktivität              o,oo3o U/ml

Erzielte Geschwindigkeit           o,o77 Ext/1o Min.

Erforderlicher Enzymeinsatz
für diese Geschwindigkeit          o,oo75 U/ml
ohne Adjuvans

Erzielte Aktivität                 25o %


B e i s p i e l    2

In o,1 M Kaliumphosphatpuffer, pH 7,o

1,5 % Tergitol NPX


| Eingesetzte Aktivität | Resultierende Geschwindigkeit |
|---|---|
| o,oo12 U/ml | o,o19 Ext/1o Min. |
| o,oo3o U/ml | o,o3o Ext/1o Min. |
| o,oo6o U/ml | o,o55 Ext/1o Min. |
| o,oo9o U/ml | o,o91 Ext/1o Min. |
| o,o12o U/ml | o,12o Ext/1o Min. |


2.1   Zusatz von o,4 Vol.-% 2,2,2-Trichloräthanol

Eingesetzte Aktivität              o,oo3 U/ml

Erzielte Geschwindigkeit           o,o46 Ext/1o Min.

Erforderlicher Enzymeinsatz
für diese Geschwindigkeit          o,oo46 U/ml
ohne Adjuvans

Erzielte Aktivität                 153 %


2.2   Zusatz von 2 Vol.-% tert.-Amylalkohol

Eingesetzte Aktivität              o,oo3 U/ml

Erzielte Geschwindigkeit           o,o53 Ext/1o Min.

Erforderlicher Enzymeinsatz
für diese Geschwindigkeit          o,oo53 U/ml
ohne Adjuvans

Erzielte Aktivität                 177 %

2.3  Zusatz von 1,4 Vol.-% 4-Methylpentanol-(2)

Eingesetzte Aktivität                    o,oo3 U/ml

Erzielte Geschwindigkeit                 o,o59 Ext/1o Min.

Erforderlicher Enzymeinsatz
für diese Geschwindigkeit                o,oo59 U/ml
ohne Adjuvans

Erzielte Aktivität                       197 %


2.4  Zusatz von 2 Vol.-% Cyclohexanol

Eingesetzte Aktivität                    o,oo3 U/ml

Erzielte Geschwindigkeit                 o,114 Ext/1o Min.

Erforderlicher Enzymeinsatz
für diese Geschwindigkeit                o,o114 U/ml
ohne Adjuvans

Erzielte Aktivität                       38o %


B e i s p i e l   3


In o,3 M Kaliumphosphatpuffer, pH 7,o

5 %o Genapol OX-1oo/5 %o Na-Caprylat


| Eingesetzte Aktivität | Resultierende Geschwindigkeit |
|---|---|
| o,oo12 U/ml | o,o1 Ext/1o Min. |
| o,oo3o U/ml | o,o23 Ext/1o Min. |
| o,oo6o U/ml | o,o38 Ext/1o Min. |
| o,oo9o U/ml | o,o55 Ext/1o Min. |
| o,o15o U/ml | o,o93 Ext/1o Min. |
| o,o24o U/ml | o,138 Ext/1o Min. |


3.1  Zusatz von 4 mM/l 3,4-Dichlorphenol (o,o65 Gew.-%)

Eingesetzte Aktivität                    o,oo3o U/ml

Erzielte Geschwindigkeit                 o,o58 Ext/1o Min.

Erforderlicher Enzymeinsatz
für diese Geschwindigkeit                o,oo93 U/ml
ohne Adjuvans

Erzielte Aktivität                       31o %

3.2   Zusatz von 1o mM/1 (ca. o,o9 Gew.-%) Phenol

Eingesetzte Aktivität                    o,oo3 U/ml

Erzielte Geschwindigkeit                 o,o64 Ext/1o Min.

Erforderlicher Enzymeinsatz
für diese Geschwindigkeit                o,o1o4 U/ml
ohne Adjuvans

Erzielte Aktivität                       347 %


3.3   Zusatz von 1 Vol.-% 4-Methylpentanol-(2)

Eingesetzte Aktivität                    o,oo3 U/ml

Erzielte Geschwindigkeit                 o,o69 Ext/1o Min.

Erforderlicher Enzymeinsatz
für diese Geschwindigkeit                o,o112 U/ml
ohne Adjuvans

Erzielte Aktivität        .              373 %


3.4   Zusatz von 2 Vol.-% Cyclohexanol

Eingesetzte Aktivität                    o,oo3 U/ml

Erzielte Geschwindigkeit                 o,o8o Ext/1o Min.

Erforderlicher Enzymeinsatz
für diese Geschwindigkeit                o,o129 U/ml
ohne Adjuvans

Erzielte Aktivität                       43o %


3.5   Zusatz von o,3 Vol.-% 2,2,2-Trichloräthanol

Eingesetzte Aktivität                    o,oo3 U/ml

Erzielte Geschwindigkeit                 o,o89 Ext/1o Min.

Erforderlicher Enzymeinsatz
für diese Geschwindigkeit                o,o144 U/ml
ohne Adjuvans

Erzielte Aktivität                       48o %


b)   Erzielung höherer Reaktionsgeschwindigkeiten unter
     vorgegebenen Bedingungen.


In vielen Fällen sind bestimmte Bedingungen, wie etwa
Puffer und Ionenstärke, sowie Art des Detergens in Reaktionsgemischen aufgrund übergeordneter Gesichtspunkte bereits vorgegeben. Solche Gründe können z. B. durch größere Empfindlichkeit eines anderen, noch im Gemisch benötigten Enzyms gegeben sein. In diesen Fällen ist die bei konstantem Cholesterinesterase-Einsatz erzielbare maximale Geschwindigkeit der Esterspaltung durch die Abhängigkeit der Esteraseaktivierung von der Art des Detergens und der Detergenskonzentration gekennzeichnet und begrenzt. Diese Abhängigkeit zeigt im allgemeinen im unteren Konzentrationsbereich an Detergens ein Optimum. Die an diesem Punkt erreichte Geschwindigkeit ist nach den oben angeführten Bedingungen nicht überbietbar und stellt in dieser Hinsicht eine Grenze des Systems dar.

Die im Beispiel nachfolgend beschriebenen erfindungsgemäßen Kombinationen sind geeignet, solche Grenzen zu übertreffen.

B e i s p i e l   4

Vorgegeben: o,1 M Tris/Weinsäurepuffer, pH 8,o
            Triton X-1oo, o,oo3 U/ml Cholesterinesterase

| %o Triton X-1oo im Ansatz | Resultierende Geschwindigkeit |
|---|---|
| o %o | o Ext/1o Min. |
| 1 %o | o,oo3 Ext/1o Min. |
| 3 %o | o,o28 Ext/1o Min. |
| 5 %o | o,o37 Ext/1o Min. |
| 7 %o | o,o37 Ext/1o Min. |
| 1o %o | o,o37 Ext/1o Min. |
| 15 %o | o,o29 Ext/1o Min. |
| Optimum | Grenze des Systems |
| 5 %o | o,o37 Ext/1o Min. |

Zusatz von Adjuvantien erfolgt bei 5 %o Triton X-1oo.

4.1 Zusatz von 2 Vol.-% tert.-Amylalkohol
Erzielte Geschwindigkeit · o,o46 Ext/1o Min.
Steigerung auf 124 %

4.2 Zusatz von o,4 Vol.-% 4-Methylpentanol-(2)
Erzielte Geschwindigkeit o,o65 Ext/1o Min.
Steigerung auf 176 %

4.3 Zusatz von 1 Vol.-% Cyclohexanol
Erzielte Geschwindigkeit o,o77 Ext/1o Min.
Steigerung auf 2o8 %

B e i s p i e l 5

Vorgegeben: o,3 M Kaliumphosphatpuffer, pH 7,o
Tergitol NPX, o,oo3 U/ml Cholesterinesterase

| %o Tergitol NPX im Ansatz | Resultierende Geschwindigkeit |
|---|---|
| o %o | o Ext/1o Min. |
| 2 %o | o Ext/1o Min. |
| 5 %o | o,o29 Ext/1o Min. |
| 1o %o | o,o42 Ext/1o Min. |
| 15 %o | o,o49 Ext/1o Min. |
| 2o %o | o,o39 Ext/1o Min. |
| Optimum | Grenze des Systems |
| 15 %o | o,o49 Ext/1o Min. |

Zusatz von Adjuvantien erfolgt bei 15 %o Tergitol NPX.

5.1 Zusatz von 2 mM/l 3,4-Dichlorphenol und 1 Vol.-%
Cyclohexanon
Erzielte Geschwindigkeit o,o58 Ext/1o Min.
Steigerung auf 118 %

5.2 Zusatz von 2 Vol.-% tert.-Amylalkohol
Erzielte Geschwindigkeit     0,066 Ext/10 Min.
Steigerung auf        135 %

5.3 Zusatz von 1,4 Vol.-% Cyclohexanol
Erzielte Geschwindigkeit     0,118 Ext/10 Min.
Steigerung auf        241 %

c)   <u>Verwendung von Puffern geringerer Ionenstärke</u>

Wie schon erwähnt, unterstützen Puffer höherer Ionenstärke die aktivierende Wirkung von Detergentien auf Cholesterinesterase. Im Rahmen der geforderten möglichst freien Wählbarkeit der Bedingungen ist es jedoch wünschenswert, nicht auf hohe Salzgehalte in der Lösung angewiesen zu sein. Das folgende Beispiel zeigt, daß erfindungsgemäß die Salzgehalte wesentlich verringert werden können.

Gegenübergestellt werden:
0,3 M Kaliumphosphatpuffer, pH 7,0
0,1 M Kaliumphosphatpuffer, pH 7,0

Durch Zusatz geeigneter Adjuvantien zum 0,1 M Puffersystem lassen sich die Geschwindigkeiten derart steigern, daß sie den höheren Vergleichswert des 0,3 M Puffersystems ohne Adjuvans erreichen und zum Teil deutlich übertreffen.

B e i s p i e l   6

15 %o Tergitol NPX, 0,003 U/ml Cholesterinesterase

6.1 Zusatz von 2 Vol.-% tert.-Amylalkohol

|     System              |     Geschwindigkeit      |
| ----------------------- | ------------------------ |
| o,1 m Puffer ohne Zusatz | o,o3o Ext/1o Min.        |
| o,3 m Puffer ohne Zusatz | o,o49 Ext/1o Min.        |
| o,1 m Puffer mit Zusatz  | o,o53 Ext/1o Min.        |

Der Vergleichswert wird übertroffen.


6.2  Zusatz von 1,4 Vol.-% 4-Methylpentanol-(2)

|     System              |     Geschwindigkeit      |
| ----------------------- | ------------------------ |
| o,1 m Puffer ohne Zusatz | o,o3o Ext/1o Min.        |
| o,3 m Puffer ohne Zusatz | o,o49 Ext/1o Min.        |
| o,1 m Puffer mit Zusatz  | o,o59 Ext/1o Min.        |

Der Vergleichswert wird übertroffen.


6.3  Zusatz von 2 Vol.-% Cyclohexanol

|     System              |     Geschwindigkeit      |
| ----------------------- | ------------------------ |
| o,1 m Puffer ohne Zusatz | o,o3o Ext/1o Min.        |
| o,3 m Puffer ohne Zusatz | o,o49 Ext/1o Min.        |
| o,1 m Puffer mit Zusatz  | o,114 Ext/1o Min.        |

Der Vergleichswert wird deutlich übertroffen.

B e i s p i e l   7

1o %o Genapol OX-1oo/1o %o Na-Caprylat
o,oo3 U/ml Cholesterinesterase

7.1  Zusatz von 4o mM (ca. o,35 Gew.-%) Phenol

|     System              |     Geschwindigkeit      |
| ----------------------- | ------------------------ |
| o,1 m Puffer ohne Zusatz | o,o17 Ext/1o Min.        |
| o,3 m Puffer ohne Zusatz | o,o53 Ext/1o Min.        |
| o,1 m Puffer mit Zusatz  | o,o53 Ext/1o Min.        |

- 19 -                                              0016946

Der Vergleichswert wird erreicht.

7.2   Zusatz von 1 Vol.-% 2,2,2-Trichloräthanol

| System | Geschwindigkeit |
|---|---|
| o,1 m Puffer ohne Zusatz | o,o17 Ext/1o Min. |
| o,3 m Puffer ohne Zusatz | o,o53 Ext/1o Min. |
| o,1 m Puffer mit Zusatz | o,o63 Ext/1o Min. |

Der Vergleichswert wird übertroffen.

7.3   Zusatz von 2 Vol.-% Cyclohexanol

| System | Geschwindigkeit |
|---|---|
| o,1 m Puffer ohne Zusatz | o,o17 Ext/1o Min. |
| o,3 m Puffer ohne Zusatz | o,o53 Ext/1o Min. |
| o,1 m Puffer mit Zusatz | o,o72 Ext/1o Min. |

Der Vergleichswert wird übertroffen.

d) Verwendung von Detergentien in geringerer Konzentration

Ist ein Detergens in der Lage, die Cholesterinesterase zu aktivieren, so muß es zur vollen Entfaltung dieser Eigenschaft in einer bestimmten Konzentration im Reaktionsgemisch vorliegen. Dieses Detergensoptimum ist wesentlich von der Art des Detergens abhängig und kann in vielen Fällen in einem ungünstig hohen Konzentrationsbereich liegen. Dadurch kann es bei anderen, für Folgereaktionen benötigten Enzymen zu Hemmungen und auch aus Gründen der Löslichkeit und der Galenik zu Schwierigkeiten kommen. Unterhalb des Detergensoptimums wird nur eine unzureichende Aktivierung beobachtet. Im Interesse einer möglichst freien Wählbarkeit der Bedingungen ist es jedoch wünschenswert, möglichst viele verschiedene Detergentien, auch solche mit höher liegendem Konzentrationsoptimum, verwenden zu können.

Das nachstehende Beispiel zeigt, daß es erfindungsgemäß möglich ist, mit weniger Detergens die Reaktionsgeschwindigkeiten des Konzentrationsoptimums zu erreichen und zu übertreffen, wodurch Detergens eingespart wird.

B e i s p i e l   8

o,3 M Kaliumphosphatpuffer, pH 7,o
Tergitol NPX, o,oo3 U/ml Cholesterinesterase

| %o Tergitol NPX im Ansatz | Resultierende Geschwindigkeit |
|---|---|
| o %o | o Ext/1o Min. |
| 2 %o | o Ext/1o Min. |
| 5 %o | o,o29 Ext/1o Min. |
| 1o %o | o,o42 Ext/1o Min. |
| 15 %o | o,o49 Ext/1o Min. |
| 2o %o | o,o39 Ext/1o Min. |

| Optimum | Vergleichswert |
|---|---|
| 15 %o | o,o49 Ext/1o Min. |

8.1   Zusatz von o,1 Vol.-% 2,2,2-Trichloräthanol

| System | Geschwindigkeit |
|---|---|
| 15 %o Tergitol ohne Zusatz | o,o49 Ext/1o Min. |
| 5 %o Tergitol ohne Zusatz | o,o29 Ext/1o Min. |
| 5 %o Tergitol mit Zusatz | o,o59 Ext/1o Min. |

Der Vergleichswert wird übertroffen.

0016946

8.2  Zusatz von 2 Vol.-% tert.-Amylalkohol

| System | Geschwindigkeit |
|---|---|
| 15 %o Tergitol ohne Zusatz | o,o49 Ext/1o Min. |
| 5 %o Tergitol ohne Zusatz | o,o29 Ext/1o Min. |
| 5 %o Tergitol mit Zusatz | o,o75 Ext/1o Min. |

Der Vergleichswert wird deutlich übertroffen.

8.3  Zusatz von 1 Vol.-% Cyclohexanol

| System | Geschwindigkeit |
|---|---|
| 15 %o Tergitol ohne Zusatz | o,o49 Ext/1o Min. |
| 5 %o Tergitol ohne Zusatz | o,o29 Ext/1o Min. |
| 5 %o Tergitol mit Zusatz | o,124 Ext/1o Min. |

Der Vergleichswert wird deutlich übertroffen.

B e i s p i e l   9

o,3 M Kaliumphosphatpuffer, pH 7,o
Genapol OX-1oo/Natriumcaprylat, o,oo3 U/ml Cholesterinesterase

| %o Genapol OX-1oo/Na-Caprylat | Geschwindigkeit |
|---|---|
| o %o | o Ext/1o Min. |
| 5 %o | o,o2o Ext/1o Min. |
| 1o %o | o,o53 Ext/1o Min. |
| 15 %o | o,o62 Ext/1o Min. |
| 2o %o | o,o66 Ext/1o Min. |
| 3o %o | o,o68 Ext/1o Min. |
| 4o %o | o,o68 Ext/1o Min. |

| Optimum | Vergleichswert |
|---|---|
| 3o %o | o,o68 Ext/1o Min. |

9.1 Zusatz von 1 Vol.-% 4-Methylpentanol-(2)

| System | Geschwindigkeit |
|---|---|
| 3o %o Genapol/Caprylat ohne Zusatz | o,o68 Ext/1o Min. |
| 5 %o Genapol/Caprylat ohne Zusatz | o,o2o Ext/1o Min. |
| 5 %o Genapol/Caprylat mit Zusatz | o,o69 Ext/1o Min. |

Der Vergleichswert wird erreicht.

9.2 Zusatz von 2 Vol.-% Cyclohexanol

| System | Geschwindigkeit |
|---|---|
| 3o %o Genapol/Caprylat ohne Zusatz | o,o68 Ext/1o Min. |
| 5 %o Genapol/Caprylat ohne Zusatz | o,o2o Ext/1o Min. |
| 5 %o Genapol/Caprylat mit Zusatz | o,o8o Ext/1o Min. |

Der Vergleichswert wird übertroffen.

9.3 Zusatz von o,3 Vol.-% 2,2,2-Trichloräthanol

| System | Geschwindigkeit |
|---|---|
| 3o %o Genapol/Caprylat ohne Zusatz | o,o68 Ext/1o Min |
| 5 %o Genapol/Caprylat ohne Zusatz | o,o2o Ext/1o Min |
| 5 %o Genapol/Caprylat mit Zusatz | o,o89 Ext/1o Min. |

Der Vergleichswert wird deutlich übertroffen.

e) Verwendung von Detergentien mit unter bestimmten Bedingungen keiner oder nur unzureichender aktivierender Wirkung

Unter der Vielzahl der zur Verfügung stehenden Detergentien erweisen sich nur wenige als geeignet, die Cholesterinesterase in ausreichendem Maße zu aktivieren. Hemmung anderer Reaktionspartner, Stabilität des Reaktionsgemisches, Konsistenz, Löslichkeit und Eindeutigkeit der
Verbindung bzw. des Präparats zählen zu den übergeordneten Gründen, die eine möglichst freie Wählbarkeit der
Detergentien wünschenswert erscheinen lassen. Das nachstehende Beispiel zeigt, daß auch Detergentien mit unter
den gegebenen Bedingungen nur geringer oder überhaupt
keiner aktivierenden Wirkung erfindungsgemäß verwendbar
werden.

B e i s p i e l  1o

o,1 M Phosphatpuffer, pH 7,o
o,oo3 U/ml Cholesterinesterase

Detergens: 6 %o Thesit
Zusatz:    1 Vol.-% n-Amylalkohol

| System | Geschwindigkeit |
|---|---|
| Detergens ohne Zusatz | o,oo3 Ext/1o Min. |
| Zusatz | o Ext/1o Min. |
| Detergens mit Zusatz | o,o29 Ext/1o Min. |

B e i s p i e l  11

o,1 M Phosphatpuffer, pH 7,o
o,oo3 U/ml Cholesterinesterase

0016946

Detergens: 6 %o Thesit
Zusatz:   1o mM/l (ca. o,16 Gew.-%) 1,7-Dihydroxy-
          naphthalin

| System | Geschwindigkeit |
|---|---|
| Detergens ohne Zusatz | o,oo3 Ext/1o Min. |
| Zusatz | o,oo5 Ext/1o Min. |
| Detergens mit Zusatz | o,o24 Ext/1o Min. |

B e i s p i e l   12

o,1 M Phosphatpuffer, pH 7,o
o,oo3 U/ml Cholesterinesterase

Detergens: 12 %o Thesit
Zusatz:   2 Vol.-% Cyclohexanol

| System | Geschwindigkeit |
|---|---|
| Detergens ohne Zusatz | o,o1o Ext/1o Min. |
| Zusatz | o,oo3 Ext/1o Min. |
| Detergens mit Zusatz | o,1o5 Ext/1o Min. |

B e i s p i e l   13

o,1 M Phosphatpuffer, pH 7,o
o,oo3 U/ml Cholesterinesterase

Detergens: 12 %o Thesit
Zusatz:   1 Vol.-% 2,2,2-Trichloräthanol

| System | Geschwindigkeit |
|---|---|
| Detergens ohne Zusatz | o,o1o Ext/1o Min. |
| Zusatz | o Ext/1o Min. |
| Detergens mit Zusatz | o,o81 Ext/1o Min. |

B e i s p i e l    14

o,1 M Tris/Weinsäure-Puffer, pH 8,o
o,oo3 U/ml Cholesterinesterase

Detergentien: 2 Vol.-%o Thesit/2 Vol.-%o Tween 2o
Zusatz:          o,4 Vol-% Hexanol-1

| System | Geschwindigkeit |
|---|---|
| Detergentien ohne Zusatz | o,o18 Ext/1o Min. |
| Zusatz | o Ext/1o Min. |
| Detergentien mit Zusatz | o,o28 Ext/1o Min. |

B e i s p i e l    15

o,1 M Phosphat-Puffer, pH 7,o
o,oo3 U/ml Cholesterinesterase

Detergentien: 5 Vol.-%o Thesit/5 Vol.-%o Tween 2o
Zusatz:          8 mM/l 3,5-Dichlorphenol

| System | Geschwindigkeit |
|---|---|
| Detergentien ohne Zusatz | o,oo5 Ext/1o Min. |
| Zusatz | o Ext/1o Min. |
| Detergentien mit Zusatz | o,o33 Ext/1o Min. |

B e i s p i e l    16

o,3 M Phosphat-Puffer, pH 7,o
o,oo3 U/ml Cholesterinesterase

Detergentien: 5 Vol.-%o Genapol OX-1oo/5 Gew.-%o Na-
                 Caprylat
Zusatz:          1o mM/l Phenol

| System | Geschwindigkeit |
|---|---|
| Detergentien ohne Zusatz | o,o2o Ext/1o Min. |
| Zusatz | o Ext/1o Min. |
| Detergentien mit Zusatz | o,o64 Ext/1o Min. |

B e i s p i e l  17

o,3 M Phosphat-Puffer, pH 7,o
o,oo3 U/ml Cholesterinesterase

Detergentien: 5 Vol.-%o Genapol OX-1oo/5 Gew.-%o Na-
              Caprylat
Zusatz:       4 mM/l (ca. o,o65 Gew.-%) 3,4-Dichlor-
              phenol

| System | Geschwindigkeit |
|---|---|
| Detergentien ohne Zusatz | o,o2o Ext/1o Min. |
| Zusatz | o Ext/1o Min. |
| Detergentien mit Zusatz | o,o58 Ext/1o Min. |

B e i s p i e l  18

o,1 M Phosphat-Puffer, pH 7,o
o,oo3 U/ml Cholesterinesterase

Detergens: 1o Vol.-%o Brij 35 (3o %-ige Lösung)
Zusatz:    4 mM/l 3,4-Dichlorphenol

| System | Geschwindigkeit |
|---|---|
| Detergens ohne Zusatz | o Ext/1o Min. |
| Zusatz | o Ext/1o Min. |
| Detergens mit Zusatz | o,o1o Ext/1o Min. |

B e i s p i e l   19

o,1 M Phosphat-Puffer, pH 7,o
o,oo3 U/ml Cholesterinesterase

Detergens: 1o Vol.-%o Brij 35 (3o %-ige Lösung)
Zusatz:      1 Vol.-% 2,2,2-Trichloräthanol

| System | Geschwindigkeit |
|---|---|
| Detergens ohne Zusatz | o Ext/1o Min. |
| Zusatz | o Ext/1o Min. |
| Detergens mit Zusatz | o,o18 Ext/1o Min. |

B e i s p i e l   2o

o,1 M Phosphat-Puffer, pH 7,o
o,oo3 U/ml Cholesterinesterase

Detergens: 2 Vol.-%o Brij 35 (3o %-ige Lösung)
Zusatz:      2 Vol.-% Cyclohexanol

| System | Geschwindigkeit |
|---|---|
| Detergens ohne Zusatz | o Ext/1o Min. |
| Zusatz | o,oo3 Ext/1o Min. |
| Detergens mit Zusatz | o,o27 Ext/1o Min. |

B e i s p i e l   21

o,1 M Phosphat-Puffer, pH 7,o
o,oo3 U/ml Cholesterinesterase

Detergens: 6 Gew.-%o Cholsäure
Zusatz:      1 Vol.-% 2,2,2-Trichloräthanol

|        System          | Geschwindigkeit        |
|------------------------|------------------------|
| Detergens ohne Zusatz  | o Ext/1o Min.          |
| Zusatz                 | o Ext/1o Min.          |
| Detergens mit Zusatz   | o,o29 Ext/1o Min.      |

B e i s p i e l   22

o,1 M Phosphat-Puffer, pH 7,o
o,oo3 U/ml Cholesterinesterase

Detergens: 6 Gew.-%o Cholsäure
Zusatz:    16 mM/l 3,5-Dichlorphenol

|        System          | Geschwindigkeit        |
|------------------------|------------------------|
| Detergens ohne Zusatz  | o Ext/1o Min.          |
| Zusatz                 | o Ext/1o Min.          |
| Detergens mit Zusatz   | o,o36 Ext/1o Min.      |

B e i s p i e l   23

o,1 M Phosphat-Puffer, pH 7,o
o,oo3 U/ml Cholesterinesterase

Detergens: 1 Gew.-%o Cholsäure
Zusätze:    4 mM/l 3,5-Dichlorphenol, 1 Vol.-% Cyclo-
            hexanol

|        System            | Geschwindigkeit        |
|--------------------------|------------------------|
| Detergens ohne Zusätze   | o Ext/1o Min.          |
| Zusätze                  | o,o1o Ext/1o Min.      |
| Detergens mit Zusätzen   | o,1o3 Ext/1o Min.      |

B e i s p i e l   24

o,1 M Tris/Weinsäure-Puffer, pH 8,o
o,oo3 U/ml Cholesterinesterase

0016946

Detergens: 3 Gew.-%o Natriumdesoxycholat

Zusatz:     o,2 Vol.-% Hexanol-1


        System                    Geschwindigkeit
Detergens ohne Zusatz            o Ext/1o Min.
Zusatz                           o Ext/1o Min.
Detergens mit Zusatz             o,o56 Ext/1o Min.


B e i s p i e l   25


o,1 M Tris/-Weinsäure-Puffer, pH 8,o

o,oo3 U/ml Cholesterinesterase


Detergens: 3 Gew.-%o Natriumdesoxycholat

Zusatz:     1 Vol.-% 2,2,2-Trichloräthanol


        System                    Geschwindigkeit
Detergens ohne Zusatz            o Ext/1o Min.
Zusatz                           o Ext/1o Min.
Detergens mit Zusatz             o,o69 Ext/1o Min.


B e i s p i e l   26


o,1 M Phosphat-Puffer, pH 7,o

o,oo3 U/ml Cholesterinesterase


Detergens: 6 Vol.-%o Genapol OX-1oo

Zusatz:     o,4 Vol.-% 2,2,2-Trichloräthanol


        System                    Geschwindigkeit
Detergens ohne Zusatz            o Ext/1o Min.
Zusatz                           o Ext/1o Min.
Detergens mit Zusatz             o,o59 Ext/1o Min.

B e i s p i e l   27

o,1 M Phosphat-Puffer, pH 7,o
o,oo3 U/ml Cholesterinesterase

Detergens: 2o Vol.-%o Tween 2o
Zusatz:    1 Vol.-% 2,2,2-Trichloräthanol

| System | Geschwindigkeit |
|---|---|
| Detergens ohne Zusatz | o,oo5 Ext/1o Min. |
| Zusatz | o Ext/1o Min. |
| Detergens mit Zusatz | o,o67 Ext/1o Min. |

<u>Anwendungsform</u>

B e i s p i e l   28

<u>Reagens zur Bestimmung von Cholesterin im Serum</u>

Rezeptur: 4,5 g/l Weinsäure
          7,5 g/l Tris
          8,7 g/l Natriumsulfat
          2oo mg/ml 4-Aminoantipyrin
          282 mg/l Phenol
          5ooo U/l Peroxidase
          43o U/l Cholesterinesterase
          31o U/l Cholesterinoxidase

Aktivierungsmittel: 2,2 g/l Thesit
                    1,3 g/l Natriumdesoxycholat
                    o,815 g/l 3,4-Dichlorphenol

Die oben getroffene Wahl der Zusätze ermöglicht die Aktivierung der Cholesterinesterase unter den durch die
Eigenschaften der anderen Enzyme gestellten Bedingungen.
Gleichzeitig ist es möglich, die Aufbewahrungsform des

0016946

Reaktionsgemisches als Eintopfreagens in fester Form zu
verwirklichen.

- 1 -

Patentansprüche

1.    Verfahren zur Aktivierung der Cholesterinesterase in ionenhaltiger Lösung durch Zusatz wenigstens eines oberflächenaktiven Mittels, dadurch g e k e n n - z e i c h n e t , daß man

a) als oberflächenaktives Mittel einen Polyäthoxyäther oder -ester oder/und eine Gallensäureverbindung und/ oder Salze von Fettsäuren mit 6 bis 1o Kohlenstoffatomen in Kombination mit

b) einem synergistisch wirkenden Alkohol, der aus den geradkettigen, verzweigten oder cyclischen aliphatischen Alkoholen mit 5 bis 12 Kohlenstoffatomen, gegebenenfalls halogensubstituierten, aromatischen Alkoholen und den mit mehreren Halogenatomen substituierten aliphatischen Alkoholen mit 1 bis 3 Kohlenstoffatomen ausgewählt ist,

zusetzt.

2.    Verfahren nach Anspruch 1, dadurch g e k e n n - z e i c h n e t , daß als Gallensäureverbindung Cholsäure, Desoxycholsäure oder ein Alkalisalz davon verwendet wird.

3. Verfahren nach Anspruch 1, dadurch g e k e n n -
z e i c h n e t , daß als Fettsäuresalz ein Alkalisalz
verwendet wird.

4. Verfahren nach einem der vorhergehenden Ansprüche,
dadurch g e k e n n z e i c h n e t , daß o,o5 bis 5 %
oberflächenaktives Mittel verwendet werden.

5. Verfahren nach einem der vorhergehenden Ansprüche,
dadurch g e k e n n z e i c h n e t , daß o,o1 bis 5 %
Alkohol verwendet werden.

6. Mittel zur Aktivierung der Cholesterinesterase in
ionenhaltiger Lösung, enthaltend wenigstens ein oberflächenaktives Mittel auf Basis eines Polyäthoxyäthers oder
-esters oder/und einer Gallensäureverbindung und/oder
Salze von Fettsäuren mit 6 bis 1o Kohlenstoffatomen,
g e k e n n z e i c h n e t durch einen Gehalt an synergistisch wirkendem Alkohol, der aus den geradkettigen Alkoholen oder verzweigtkettigen oder cyclischen
aliphatischen Alkoholen mit 5 bis 12 Kohlenstoffatomen,
gegebenenfalls halogensubstituierten, aromatischen Alkoholen, halogensubstituierten aliphatischen Alkoholen
mit 1 bis 3 Kohlenstoffatomen ausgewählt ist.

7. Mittel nach Anspruch 6, dadurch g e k e n n -
z e i c h n e t , daß es o,o5 bis 5 %, bezogen auf die
Enzymlösung, Detergens enthält.

8. Mittel nach Anspruch 7, dadurch g e k e n n -
z e i c h n e t , daß es o,1 bis 3 % oberflächenaktives Mittel enthält.

9. Mittel nach einem der Ansprüche 6 bis 8, dadurch
g e k e n n z e i c h n e t , daß es o,o1 bis 5 % Alkohol enthält.

10.    Mittel nach Anspruch 9, dadurch  g e k e n n -
z e i c h n e t , daß es 0,05 bis 3 % Alkohol enthält.

0016946

1/1

FIG.1

FIG.2
Ermittlung der Geschwindigkeit der Esterspaltung
in  Extinktionsänderung pro Zeiteinheit

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | <u>FR - A - 2 263 516</u> (ABBOTT LAB.)<br><br>* Seite 6, Zeilen 9-38; Patentanspruch 10 *<br><br>-- | 1,2,4-10 | C 12 N 9/18<br>C 12 N 9/96<br>C 12 Q 1/60 |
| X | <u>DE - A - 2 612 725</u> (BOEHRINGER MANNHEIM)<br><br>* Seite 4, Zeile 17 - Seite 5, Zeile 30; Beispiele 1,2; Patentansprüche 1,4 *<br><br>-- | 1,4-10 | |
| P,E | <u>EP - A - 0 004 857</u> (BOEHRINGER MANNHEIM)<br><br>* Beispiel 38 *<br><br>---- | 1-10 | **RECHERCHIERTE SACHGEBIETE (Int. Cl. 3)**<br><br>C 12 N 9/18<br>C 12 N 9/16<br>C 12 N 9/14<br>C 12 N 9/96<br>C 12 Q 1/00<br>C 12 Q 1/60 |

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 01-07-1980 | DESCAMPS |

EPA form 1503.1  06.78